# EUROPÄISCHE PATENTSCHRIFT

(11) **EP 0 733 619 B1**
(45) Veröffentlichungstag und Bekanntmachung des Hinweises auf die Patenterteilung: **28.10.1998**
(21) Anmeldenummer: 96103710.8
(22) Anmeldetag: 09.03.1996
(51) Int. Cl.: C07C 403/24

(54) **Verfahren zur Herstellung von Astaxanthin**
Process for the preparation of astaxanthin
Procédé pour la préparation d'astaxanthine

(30) Priorität: 18.03.1995 DE 19509955
(43) Veröffentlichungstag der Anmeldung: 25.09.1996
(73) Patentinhaber: BASF Aktiengesellschaft, 67063 Ludwigshafen (DE)
(72) Erfinder: Krause, Wolfgang, Dr., 68782 Brühl (DE); Henrich, Klaus, 67454 Hassloch (DE); Paust, Joachim, Dr., 67141 Neuhofen (DE); Ernst, Hansgeorg, Dr., 67346 Speyer (DE)

(56) Entgegenhaltungen:
- EP-A- 0 005 748
- EP-A- 0 283 979
- DE-A- 2 653 838
- FR-A- 1 163 369
- FR-A- 1 383 944

## Beschreibung

Die Erfindung betrifft ein verbessertes Verfahren zur Herstellung des C₄₀-Carotinoids Astaxanthin durch eine doppelte Wittig-Reaktion eines 3-Methyl-5-(2,6,6-trimethyl-3-oxo-4-hydroxy-1- cyclohexen-1-yl)-2,4-pentadienyl-triphenylphosphoniumsalzes(AstaC₁₅-triphenylphosphoniumsalz) mit 2,7-Dimethyl-2,4,6- octatrien-1,8-dial (C₁₀-Dial).

Astaxanthin ist ein zum Färben von Lebensmitteln, Lachsen und Forellen sehr begehrter natürlicher Farbstoff. Dementsprechend sind zahlreiche Methoden zur Isolierung oder Synthese von Astaxanthin bekannt. So ist zum Beispiel die Isolierung von Astaxanthin durch Extraktion von Crustaceenschalen aus WO-A 86/6082 bekannt. Weiterhin kann Astaxanthin durch fermentative Prozesse (vgl. Biotechnol. Letters 10 (1988), 609-614 oder aus Mikroalgen (vgl. WO-A 89/1977 und EP 329 754) gewonnen werden. Diese Methoden haben jedoch entscheidende Nachteile. Zum einen liegt Astaxanthin in der Natur nur in sehr geringer Konzentration vor und muß daher durch aufwendige Prozesse isoliert werden. Andererseits werden nur unbefriedigende Ausbeuten erzielt. Außerdem sind die großen Mengen an benötigten Hilfsstoffen und an anfallenden nicht verwertbaren Abfallstoffen ökologisch und ökonomisch nicht akzeptabel.

Von den Verfahren zur synthetischen Herstellung von Astaxanthin sei die Oxidation von Canthaxanthin-bis-silyl-enolethern mit Percarbonsäuren und anschließende Hydrolyse (vgl. EP 101 597) genannt. Nachteilig an diesem Verfahren sind die nur mäßigen Ausbeuten und Reinheiten an Astaxanthin, unvollkommene Umsätze und unerwünschte Nebenprodukte, wie Adonirubin.

Aus EP-440 037 ist eine weitere Methode zur Oxidation von Canthaxanthin-enolaten bekannt. Aber auch dieses Verfahren ist für eine technische Herstellung nicht geeignet, da zur Darstellung der Enolate teure Basen, wie die Salze von Hexamethyldisilazan, und sehr tiefe Temperaturen notwendig sind und als Oxidationsmittel das schlecht verfügbare und sicherheitstechnisch brisante Phenylsulfonyloxaziridin eingesetzt wird. Zudem ist auch bei diesem Verfahren der Umsatz nur unvollständig.

Aus EP 05 749 ist ein Verfahren zur Herstellung von Astaxanthin durch Wittig-Reaktion eines an der Hydroxygruppe in 4-Stellung des Asta-C₁₅-triarylphosphoniumsalzes acylierten Bausteins mit C₁₀-Dial und anschließende Hydrolyse bekannt. Als Lösungsmittel für diese Wittig-Reaktion wird u.a. Isopropanol genannt. Nachteilig an diesem Verfahren ist, daß in die C₁₅-Triphenylphosphoniumsalze Schutzgruppen eingeführt und wieder abgespalten werden müssen, sowie die erzielten mäßigen Ausbeuten.

In EP 057 48A2 wird die Wittig-Reaktion von Asta-C₁₅-Triarylphosphoniumsalzen selbst mit C₁₀-Dial beschrieben. Als geeignete Lösungsmittel werden Halogenkohlenwasserstoffe, wie Methylenchlorid und Chloroform genannt. Nachteilig an diesem Verfahren ist, daß man sowohl als Lösungsmittel als auch für die Aufarbeitung durch Extraktion große Mengen an Halogenkohlenwasserstoffen benötigt, die bekanntermaßen bedenkliche toxikologische Eigenschaften aufweisen und eine technisch aufwendige Rückgewinnung erfordern.

Weiterhin wird in mehreren Publikationen in Helv. Chim Acta 64 (1981; vgl. Seiten 2405-18; Seiten 2436-46 und Seiten 2447-62) die Umsetzung von Asta-C₁₅-Triarylphosphoniumhalogeniden mit dem C₁₀-Dial nach Wittig beschrieben. In allen beschriebenen Wittig-Reaktionen wird Methylenchlorid als Lösungsmittel für die Phosphoniumsalze und den C₁₀-Dialdehyd eingesetzt und Methylenchlorid bei der Aufarbeitung und Reinigung eingesetzt. Nachteilig hieran ist, daß Methylenchlorid als sehr leicht flüchtiger toxikologisch bedenklicher Halogenkohlenwasserstoff bei einem technischen Verfahren einen beträchtlichen apparativen Aufwand erfordert und außerdem Methylenchlorid am Astaxanthin angelagert werden kann, wodurch chlorhaltige Produkte erhalten würden.

Es war daher die Aufgabe der Erfindung, das Verfahren zur Herstellung von Astaxanthin aus einem Asta-C₁₅-Triarylphosphoniumsalz und dem C₁₀-Dialdehyd in einer Wittig-Reaktion so zu verbessern, daß man die Umsetzung auf technisch einfache Weise auch ohne Einsatz chlorierter Kohlenwasserstoffe bzw. ohne Einsatz schwierig aufzuarbeitender Lösungsmittelgemische durchführen kann.

Es wurde nun überraschenderweise gefunden, daß man Astaxanthin auf technisch einfache Weise und in sehr guten Ausbeuten herstellen kann, wenn man sowohl für die Wittig-Reaktion als auch für die Aufarbeitung nur Methanol, Ethanol oder Methanol und Ethanol im Gemisch mit Wasser verwendet.

Zwar sind aus der Literatur auch schon Carotinoid-Synthesen in reinem Alkanol bekannt (vgl. DE 1 068 703 und DE 954 247), jedoch wurden dort β-Ionylidenethyltriphenylphosphoniumsalze zur Synthese von β-Carotin eingesetzt. Die Löslichkeits- und Polaritätsunterschiede der Edukte und Produkte sind beim Vergleich des unsubstituierten β-Carotins mit dem hochsubstituierten Astaxanthin beträchtlich und lassen daher keinen Analogieschluß zu. Das Strukturelement alpha-Hydroxy-ketogruppe im Astaxanthin ist ungleich reaktiver als reine Polyene und neigt zu zahlreichen Nebenreaktionen im basischen und sauren Medium wie z.B. der Bildung von Semiastacin, Astacin und Diosphenolen (vgl. Helv. Chim. Acta 64 (1981), Seiten 2436-46 und O. Isler "Carotenoids", Birkhäuser-Verlag (1971), Seiten 140 und 141).

In DE 2 653 838 werden bei der Synthese von 15,15'-Dehydroastaxanthin zwar auch Alkanole erwähnt, es wird jedoch - neben Dichlormethan und Dimethylformamid - nur Isopropanol expressis verbis genannt. Außerdem wird das gewünschte Produkt zusätzlich mit Methylenchlorid extrahiert und aus Chloroform/Methanol oder Pyridin/Wasser umkristallisiert. Dieses zeigt, daß eine effiziente Gewinnung des Produktes ohne Verwendung von insbesondere chlorierten Kohlenwasserstoffen für Astaxanthin-Derivate für unmöglich gehalten wurde.

Bei Verwendung von Isopropanol oder Isobutanol als Lösungsmittel für die Wittig-Reaktion werden zudem nur unbefriedigende Ausbeuten an Astaxanthin erzielt (siehe auch Vergleichsbeispiele 7 und 8). Umso erstaunlicher war es nun zu finden, daß Astaxanthin oder Astaxanthin-Derivate mit großem Vorteil in reinem Methanol oder Ethanol oder Methanol- oder Ethanol-Wassergemischen in hoher Ausbeute und Selektivität dargestellt werden können.

Gegenstand der Erfindung ist daher ein Verfahren zur Herstellung von Astaxanthin der Formel I 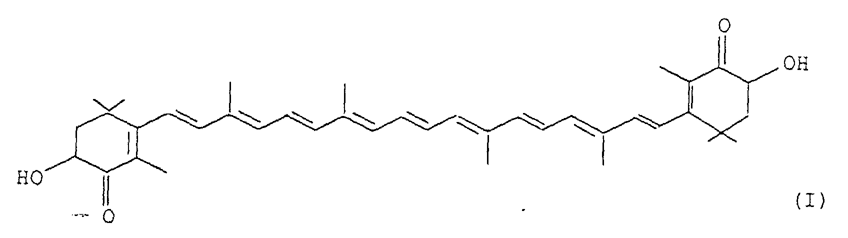 durch Umsetzen von 2 Mol des Triphenylphosphoniumsalzes der allgemeinen Formel II 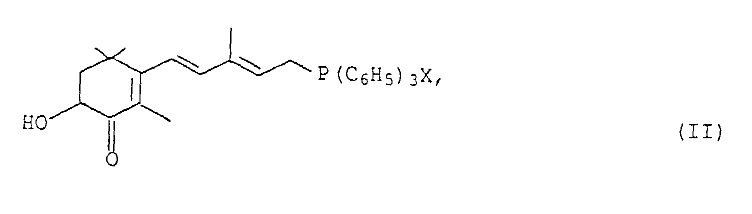 in der X für Chlor, Brom oder den (HSO₄)⁻-Rest, vorzugsweise für Brom steht, in einer Wittig-Reaktion mit einem Mol des C₁₀-Dialdehyds der Formel III 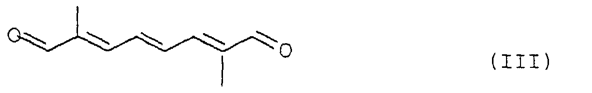 das dadurch gekennzeichnet ist, daß man
A. die Ausgangsverbindungen der Formeln II und III in Methanol, Ethanol oder einem Gemisch aus Methanol oder Ethanol mit Wasser suspendiert, die Suspension auf Temperaturen von 30 bis 80°C, vorzugsweise 40 bis 60°C erwärmt, bis sich eine homogene Lösung gebildet hat,
B. die erhaltene homogene Lösung bei Temperaturen von 20 bis 40°C mit 1 bis 1,2, vorzugsweise 1 bis 1,1 Mol, pro Mol Triphenylphosphoniumsalz, einer Base versetzt,
C. nach Beendigung der Umsetzung dem Reaktionsgemisch gegebenenfalls die für eine Ausfällung des Astaxanthins noch notwendige Menge Wasser zufügt und
D. das erhaltene E/Z-Isomerengemisch von Astaxanthin gewünschtenfalls durch Erhitzen in einem C₁- bis C₄-Alkanol, in Hexan, Heptan, Wasser oder Gemischen dieser Lösungsmittel unter Rückfluß oder im geschlossenen Gefäß auf Temperaturen von 60 bis 120°C, vorzugsweise 80 bis 100°C, in das all-(E)-Isomere isomerisiert bzw. in das all-(E)-Isomere isomerisiert und reinigt.

Besonders vorteilhaft gestaltet sich das Verfahren, wenn man das erhaltene Astaxanthin durch Erhitzen in Methanol oder Ethanol im geschlossenen Gefäß auf Temperaturen von 60 bis 120°C, vorzugsweise 80 bis 100°C, isomerisiert.

Zur Durchführung der erfindungsgemäßen Umsetzung verwendet man im allgemeinen soviel Methanol oder Ethanol, daß die Konzentration der Triphenylphosphoniumhalogenide im Alkanol 0,1 bis 3 Mol, vorzugsweise 0,3 bis 1 Mol pro Liter Alkanol beträgt.

Zur vollständigen Auflösung der Edukte der Formeln II und III wird im allgemeinen so vorgegangen, daß man sie in beliebiger Reihenfolge in das Alkanol einträgt und die Suspension auf 30 bis 40°C, vorzugsweise 50 bis 60°C erhitzt. Der Gehalt an Wasser im Reaktionsgemisch beträgt hierbei mit Vorteil 0 bis 25 Gew.%.

In die entstandene Lösung wird im allgemeinen bei Temperaturen von 0 bis 80°C, vorzugsweise 10 bis 30°C, langsam die Base eingetragen.

Als für Wittig-Reaktionen geeignete Basen seien genannt: Lösungen von Alkali- oder Erdalkalialkoxiden oder Alkali- oder Erdalkalihydroxiden in Methanol oder Ethanol, Alkali- oder Erdalkalihydroxide, Ammoniak, Triethylamin sowie Alkali- oder Erdalkalicarbonate. Besonders vorteilhaft gestaltet sich das Verfahren, wenn man eine wäßrige Lösung von NaOH oder KOH, eine methanolische Natriummethoxidlösung oder eine ethanolische Natriumethoxidlösung verwendet.

Die Reaktionszeiten für das Lösen der Edukte sowie Eintragen der Basen und Umsetzung betragen im allgemeinen 0,5 bis 12 Stunden, vorzugsweise 1 bis 8 Stunden.

Zur Aufarbeitung des Reaktionsgemisches wird dieses, sofern es noch nicht die für die Ausfällung des Astaxanthins notwendige Menge Wasser enthält, mit Wasser versetzt.

Es ist vorteilhaft für die Isolierung, wenn das Reaktionsgemisch etwa 0,1 bis 10, vorzugsweise 0,5 bis 2 Volumenteile Wasser pro Volumenteil Alkanol enthält.

Es ist besonders vorteilhaft, wenn man die Isolierung des Astaxanthins bei Temperaturen von 40 bis 80°C, vorzugsweise 50 bis 60°C, durchführt.

Das auskristallisierte Astaxanthin wird durch Filtration oder Zentrifugation isoliert und mit Methanol, Ethanol und/oder Gemischen dieser Alkanole mit Wasser gewaschen.

Zur Überführung des erhaltenen E/Z-Isomerengemisches in das besonders begehrte all-(E)-konfigurierte Astaxanthin, wird dieses im allgemeinen einer thermischen Behandlung unterworfen. Die thermische Isomerisierung des erfindungsgemäß erhaltenen Astaxanthins wird mit Vorteil so durchgeführt, daß man das noch wasserfeuchte, alkanolfeuchte oder wasser-alkanol-feuchte Kristallisat in einem C₁- bis C₄-Alkanol, vorzugsweise in Methanol oder Ethanol, in Heptan, Wasser oder Gemischen dieser Lösungsmittel suspendiert und entweder unter Rückfluß unter Normaldruck oder im geschlossenen Gefäß unter dem sich einstellenden Druck auf Temperaturen von 60 bis 120°C, vorzugsweise 70 bis 100°C, erhitzt.

Mit besonderem Vorteil gelingt das erfindungsgemäße Verfahren, wenn man das Astaxanthin enthaltende Reaktionsgemisch vor der thermischen Isomerisierung mit Essigsäure neutralisiert, da die Sauerstoff-Funktionen im Ring des Astaxanthins in alkalischem Medium empfindlicher sind.

Die Isomerisierungszeiten betragen je nach Reaktionstemperatur 0,5 bis 16 Stunden, insbesondere 1 bis 10 Stunden.

Mit Hilfe des erfindungsgemäßen Verfahrens kann das als Nahrungsmittelfarbstoff begehrte Astaxanthin in Ausbeuten von bis zu 88 % der Theorie und mit Selektivitäten in bezug auf die all-(E)-Konfiguration von bis zu 99 % erhalten werden.

### Beispiele 1 bis 8

### (Beispiel 7 und 8 sind Vergleichsbeispiele)

Jeweils 132,5 g (0,228 mol) 3-Methyl-(2,6,6-trimethyl-3-oxo-4-hydroxy-1-cyclohexen-1-yl)-2,4-pentadienyl-triphenylphosphoniumbromid (C15-Asta-triphenylphosphoniumbromid) und 16,6 g (0,1 mol) 2,7-Dimethyl-2,4,6-octatrien-1,8-dial (99 gew.-%ig; all-E/4Z=2/1) wurden bei 25°C in 500 ml des aus der folgenden Tabelle genannten Alkanols suspendiert. Es wurde auf die aus der Tabelle ersichtliche Temperatur erhitzt, wobei eine homogene Lösung entstand. Nach kurzem Rühren bei 50°C wurde auf etwa 25°C abgekühlt und innerhalb der aus der Tabelle ersichtlichen Zeit bei der aus der Tabelle ersichtlichen Temperatur die aus der Tabelle ersichtliche Base zugefügt.

Anschließend wurde bei der aus der Tabelle ersichtlichen Temperatur 2 Stunden (h) nachgerührt. Zur Aufarbeitung wurden dem Reaktionsgemisch bei 25°C die aus der Tabelle ersichtliche Menge Wasser zugefügt, das Reaktionsgemisch noch für die aus der Tabelle ersichtliche Zeit bei der aus der Tabelle ersichtlichen Temperatur nachgerührt und anschließend die gebildete Astaxanthin-Kristallsuspension abgesaugt und mit einem 60 volumen-%igen Alkanol-Wasser-Gemisch sowie mit warmem Wasser nachgewaschen.

Zur Isomerisierung wurden die wasserfeuchten Kristalle in 200 ml des aus der Tabelle ersichtlichen Alkanols gelöst und mehrere Stunden unter Rückfluß zum Sieden erhitzt (Beispiele 1, 3 und 5) oder im Druckgefäß auf Temperaturen von 95 bis 100°C erhitzt (Beispiele 2, 4 oder 6). Anschließend kühlte man auf 25°C ab, saugte ab und, wusch das Kristallisat mit dem entsprechenden Alkanol nach. Die Trocknung der Astaxanthin-Kristalle erfolgte bei 50°C und 1 mbar bis zur Gewichtskonstanz.

Die erzielten Ausbeuten an Astaxanthin wurden in g sowie in % der Theorie, bezogen auf eingesetzten Cio-Dialdehyd in der Tabelle angegeben. Auch der erzielte all-E-Gehalt sowie der Schmelzpunkt Fp des jeweils erhaltenen Astaxanthins sind in der Tabelle angegeben.

Die Beispiele 7 und 8 mit Verwendung von Isopropanol bzw. Isobutanol sind nur Vergleichsbeispiele.

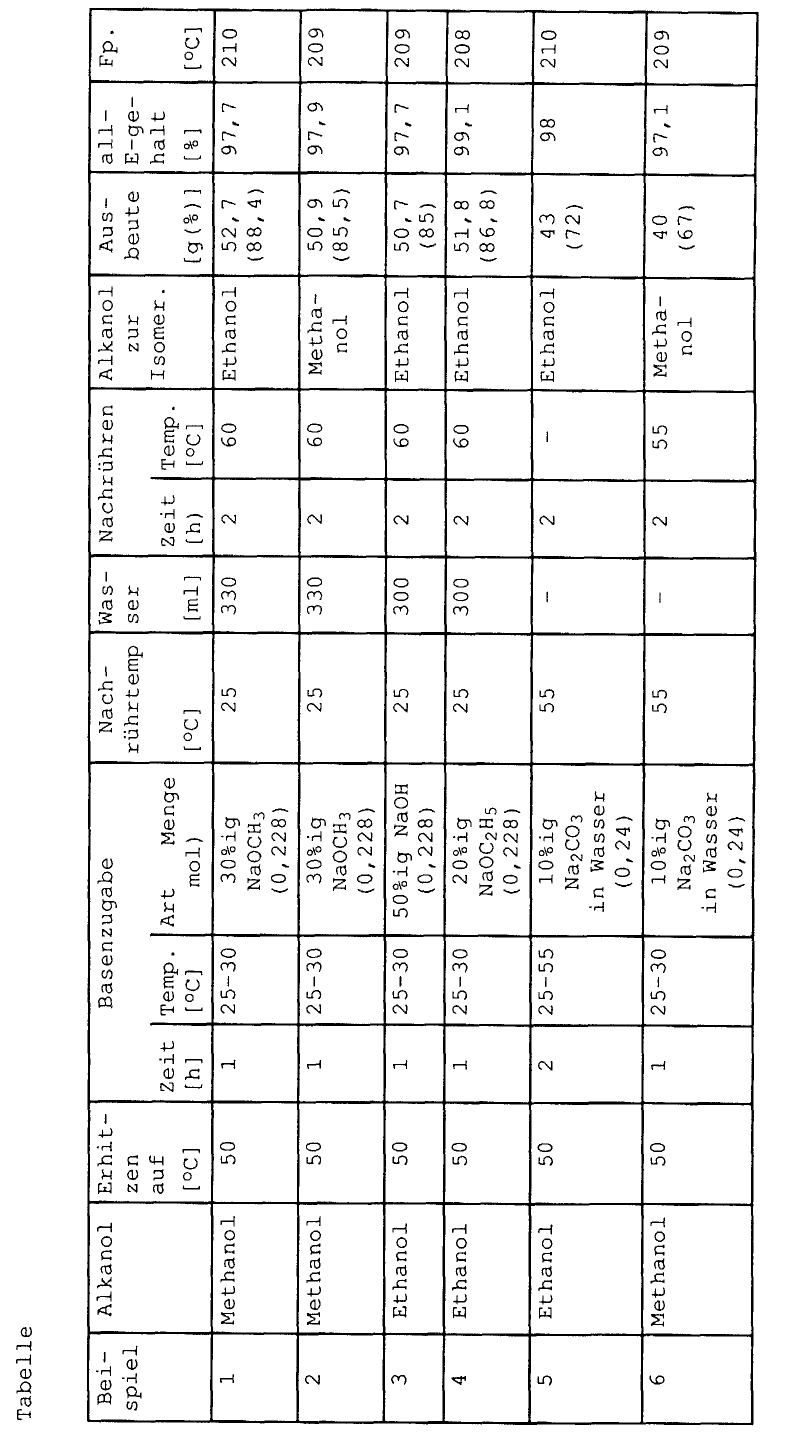

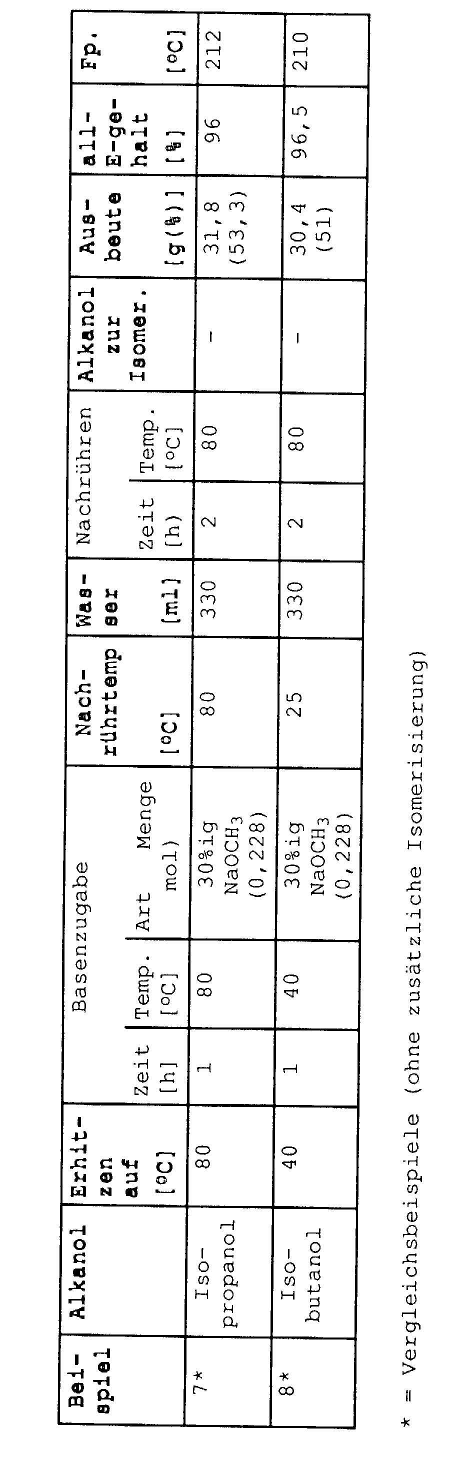

### Beispiel 9

132,5 g (0,228 mol) C₁₅-Asta-triphenylphosphoniumchlorid (99 %ig) 5 und 16,6 g (0,1 mol) Cio-Dialdehyd (99 %ig; all E/4-Z=2/1) wurden bei 25°C in 500 ml Ethanol suspendiert. Es wurde auf 50°C erhitzt, wobei eine homogene Lösung entstand. Nach kurzem Rühren bei 50°C wurde auf 25°C abgekühlt und innerhalb von 1 h bei 25-30°C als Base 50 %ige wäßrige NaOH (18,3 g = 0,228 mol) zugegeben.
Die Nachrührzeit bei 25°C betrug 2 h.
Zur Aufarbeitung wurden bei 25°C 300 ml Wasser zugegeben. Man erwärmte auf 60°C. Es wurde bei 60°C nachgerührt. Die Kristallsuspension von Astaxanthin wurde danach abgesaugt und nachgewaschen. Die Waschungen erfolgten mit einem 60 vol.-%igen Ethanol/Wassergemisch und mit warmem Wasser.

Zur Isomerisierung wurden die wasserfeuchten Kristalle von E/Z-Astaxanthin in 200 ml Ethanol suspendiert und mehrere h unter Rückfluß zum Sieden erhitzt. Man kühlte auf 25°C ab, saugte ab und wusch das Kristallisat mit Ethanol nach. Die Trocknung erfolgte bei 50°C/1 mbar bis zur Gewichtskonstanz.

Ausbeute: 46,8 g Astaxanthin (entsprechend 78,5 % bezogen auf C₁₀-Dialdehyd); gemäß HPLC betrug der all-E-Gehalt 97,1 %; Fp. 211°C.

### Beispiel 10

132,5 g (0,228 mol) C₁₅-Asta-triphenylphosphoniumchlorid (99 %ig) und 16,6 g (0,1 mol) C₁₀-Dialdehyd (99 %ig; all E/4-Z=2/1) wurden bei 25°C in 500 ml Ethanol suspendiert. Es wurde auf 50°C erhitzt, wobei eine homogene Lösung entstand. Nach kurzem Rühren bei 50°C wurde auf 25°C abgekühlt und innerhalb von 1 h bei 25-30°C als Base eine 20 %ige Lösung von NaOEt (77,6 g = 0,228 mol) in Ethanol zugegeben.
Die Nachrührzeit bei 25°C betrug 2 h.
Zur Aufarbeitung wurden bei 25°C 300 ml Wasser zugegeben. Man erwärmte auf 60°C. Es wurde bei 60°C nachgerührt. Die Kristallsuspension von Astaxanthin wurde danach abgesaugt und nachgewaschen.
Die Waschungen erfolgten mit einem 60 vol.-%igen Ethanol/Wassergemisch und mit warmem Wasser.

Zur Isomerisierung wurden die wasserfeuchten Kristalle von E/Z-Astaxanthin in 200 ml Ethanol suspendiert und mehrere h im geschlossenen Gefäß bei 100°C unter einem Eigendruck von ca. 1,5 bar unter Rückfluß zum Sieden erhitzt. Man kühlte auf 25°C ab, saugte ab und wusch das Kristallisat mit Ethanol nach. Die Trocknung erfolgte bei 50°C/1 mbar bis zur Gewichtskonstanz.

Ausbeute: 50,7 g Astaxanthin (entsprechend 85 %, bezogen auf den C₁₀-Dialdehyd); gemäß HPLC betrug der all-E-Gehalt 97 %; Fp. 211-213°C.

### Beispiel 11

132,5 g (0,228 mol) C₁₅-Asta-triphenylphosphoniumchlorid (99 %ig) und 16,6 g (0,1 mol) C₁₀-Dialdehyd (99 %ig; all E/4-Z=2/1) wurden bei 25°C in 500 ml Ethanol suspendiert. Es wurde auf 50°C erhitzt, wobei eine homogene Lösung entstand. Nach kurzem Rühren bei 50°C wurde auf 25°C abgekühlt und innerhalb von 1 h bei 25-30°C als Base eine 20 %ige Lösung von NaOEt (77,6 g = 0,228 mol) in Ethanol zugegeben.
Die Nachrührzeit bei 25°C betrug 2 h. Anschließend wurde das Reaktionsgemisch mit 3,06 g (0,051 mol) Eisessig versetzt.
Zur Aufarbeitung wurden bei 25°C 300 ml Wasser zugegeben. Man erwärmte auf 60°C. Es wurde bei 60°C nachgerührt. Die Kristallsuspension von Astaxanthin wurde danach abgesaugt und nachgewaschen.
Die Waschungen erfolgten mit einem 60 vol.-%igen Ethanol/Wassergemisch und mit warmem Wasser.

Zur Isomerisierung wurden die wasserfeuchten Kristalle von E/Z-Astaxanthin in 200 ml Ethanol suspendiert und 16 h bei 100°C unter einem Eigendruck von ca. 1,5 bar unter Rückfluß zum Sieden erhitzt. Man kühlte auf 25°C ab, saugte ab und wusch das Kristallisat mit Ethanol nach. Die Trocknung erfolgte bei 50°C/1 mbar bis zur Gewichtskonstanz.

Ausbeute: 52,8 g (88,5 %, bezogen auf C₁₀-Dialdehyd); gemäß HPLC betrug der all-E-Gehalt 96 %; Fp. 212-214°C.

## Patentansprüche

1. Verfahren zur Herstellung von Astaxanthin der Formel I 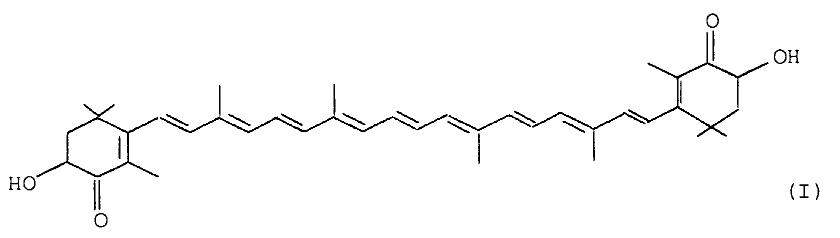 durch Umsetzen von 2 Mol des Triphenylphosphoniumsalzes der allgemeinen Formel II 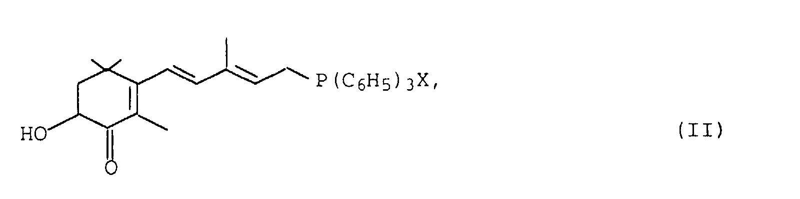 in der X für Chlor, Brom oder den (HSO₄)⁻-Rest steht, in einer Wittig-Reaktion mit einem Mol des C₁₀-Dialdehyds der Formel III 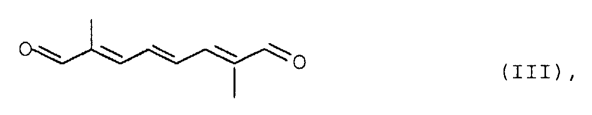 dadurch gekennzeichnet, daß man
A. die Ausgangsverbindungen der Formeln II und III in Methanol, Ethanol oder einem Gemisch aus Methanol oder Ethanol mit Wasser suspendiert, die Suspension auf Temperaturen von 30 bis 80°C erwärmt bis sich eine homogene Lösung gebildet hat,
B. die erhaltene homogene Lösung bei Temperaturen von 20 bis 40°C mit etwa 1 Mol pro Mol Triphenylphosphoniumsalz einer für Wittig-Reaktionen üblichen Base versetzt,
C. nach Beendigung der Umsetzung dem Reaktionsgemisch gegebenenfalls die für eine Ausfällung des Astaxanthins noch notwendige Menge Wasser zufügt und das auskristallisierte Astaxanthin isoliert und
D. das erhaltene E/Z-Isomerengemisch von Astaxanthin gewünschtenfalls durch Erhitzen in einem C₁- bis C₄-Alkanol, in Hexan, Heptan, Wasser oder Gemischen eines oder mehrerer dieser Lösungsmittel unter Rückfluß unter Normaldruck oder im geschlossenen Gefäß auf Temperaturen von 60 bis 120°C, isomerisiert bzw. isomerisiert und reinigt.

2. Verfahren gemäß Anspruch 1, dadurch gekennzeichnet, daß man ein Triphenylphosphoniumsalz der allgemeinen Formel II, in der X für Brom steht, mit dem C₁₀-Dialdehyd der Formel III umsetzt.

3. Verfahren gemäß Anspruch 1, dadurch gekennzeichnet, daß man im Reaktionsschritt B die homogene Lösung der Ausgangsverbindungen mit einer wässrigen Lösung von Natriumhydroxid oder Kaliumhydroxid, einer methanolischen Natriummethoxidlösung oder einer ethanolischen Natriumethoxidlösung versetzt.

4. Verfahren gemäß Anspruch 1, dadurch gekennzeichnet, daß man das erhaltene Astaxanthin enthaltende Reaktionsgemisch vor der thermischen Isomerisierung gemäß D. mit Essigsäure neutralisiert.

5. Verfahren gemäß Anspruch 1, dadurch gekennzeichnet, daß man das erhaltene Astaxanthin durch Erhitzen in Methanol oder Ethanol im geschlossenen Gefäß unter Eigendruck des Lösungsmittels auf Temperaturen von 80 bis 100°C isomerisiert bzw. isomerisiert und reinigt.

## Claims

1. A process for the preparation of astaxanthin of the formula I 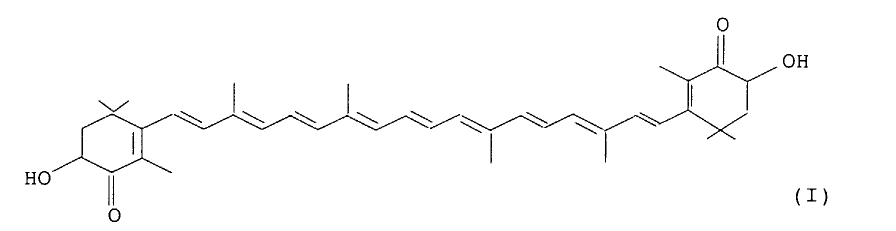 by reaction of 2 mol of the triphenylphosphonium salt of the general formula II 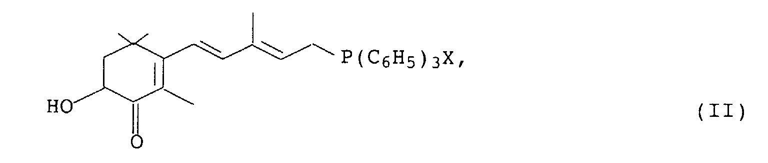 where X is chlorine, bromine or HSO₄⁻, in a Wittig reaction with one mol of the C₁₀-dialdehyde of the formula III 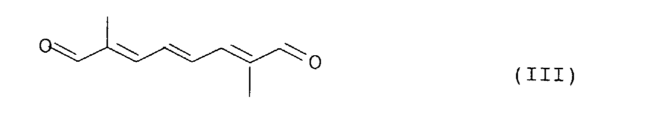 wherein
A. the starting compounds of the formulae II and III are suspended in methanol, ethanol or a mixture of methanol or ethanol with water, and the suspension is heated to from 30 to 80°C until a homogeneous solution has formed,
B. about 1 mol of a base which is conventional for Wittig reactions is added per mol of triphenylphosphonium salt to the resulting homogeneous solution at from 20 to 40°C,
C. after the reaction is complete, where appropriate, the amount of water necessary to precipitate the astaxanthin is added to the reaction mixture, and the crystallized astaxanthin is isolated, and
D. the resulting E/Z isomer mixture of astaxanthin is, if desired, isomerized, or isomerized and purified, by heating in a C₁-C₄-alkanol, in hexane, heptane, water or mixtures of one or more of these solvents to from 60 to 120°C under reflux under atmospheric pressure or in a closed vessel.

2. A process as claimed in claim 1, wherein a triphenylphosphonium salt of the general formula II where X is bromine is reacted with the C₁₀-dialdehyde of the formula III.

3. A process as claimed in claim 1, wherein in step B an aqueous solution of sodium hydroxide or potassium hydroxide, a methanolic sodium methoxide solution or an ethanolic sodium ethoxide solution is added to the homogeneous solution of the starting compounds.

4. A process as claimed in claim 1, wherein the resulting astaxanthin-containing reaction mixture is neutralized with acetic acid before the thermal isomerization according to D.

5. A process as claimed in claim 1, wherein the resulting astaxanthin is isomerized, or isomerized and purified, by heating in methanol or ethanol to from 80 to 100°C in a closed vessel under the autogenous pressure of the solvent.

## Revendications

1. Procédé de préparation d'astaxanthine de formule I 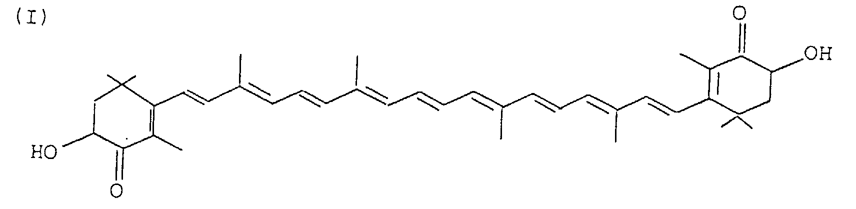 par mise en réaction de deux moles de sel de triphénylphosphonium, de formule générale II 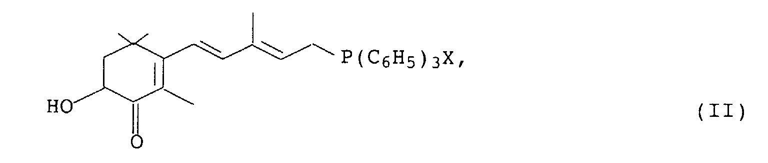 dans laquelle X est le chlore, le brome ou le résidu (HSO₄)⁻, dans une réaction de Wittig, avec une mole de dialdéhyde en C₁₀ de formule III 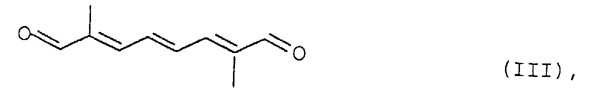 caractérisé par le fait que :
A. on met en suspension les compositions initiales des formules II et III dans du méthanol, de l'éthanol ou dans un mélange de méthanol ou d'éthanol avec de l'eau, on chauffe la suspension à des températures allant de 30 à 80°C, jusqu'à ce que se soit constituée une solution homogène,
B. on mélange la solution homogène obtenue, à des températures de 20 à 40°C, avec environ 1 mole par mole de sel de triphénylphosphonium d'une base usuelle pour des réactions de Wittig,
C. après achèvement de la conversion, on ajoute au mélange de réaction, le cas échéant, la quantité d'eau encore nécessaire pour obtenir une précipitation de l'astaxanthine et on isole l'astaxanthine qui s'est séparée avec cristallisation, et
D. on isomérise, respectivement on isomérise et on purifie, le mélange d'isomères E/Z obtenu d'astaxanthine, si on le souhaite par chauffage, dans un alcanol en C₁ à C₄, dans de l'hexane, de l'heptane, de l'eau ou des mélanges d'un ou plusieurs de ces solvants avec reflux, à une pression normale ou bien dans un récipient fermé, à des températures de 60 à 120°C.

2. Procédé selon la revendication 1, caractérisé par le fait qu'on fait réagir un sel de triphénylphosphonium de formule générale II, dans laquelle X est le brome, avec le dialdéhyde en C₁₀ de formule III.

3. Procédé selon la revendication 1, caractérisé par le fait que, à l'étape de réaction B, on mélange la solution homogène des composés de départ avec une solution aqueuse d'hydroxyde de sodium ou d'hydroxyde de potassium, avec une solution de méthoxyde de sodium méthanolique ou bien une solution de méthoxyde de sodium éthanolique.

4. Procédé selon la revendication 1, caractérisé par le fait que l'on neutralise à l'acide acétique un mélange de réaction contenant l'astaxanthine obtenue, avant l'isomérisation thermique selon D.

5. Procédé selon la revendication 1, caractérisé par le fait qu'on isomérise, respectivement on isomérise et on purifie, l'astaxanthine obtenue par chauffage dans du méthanol ou de l'éthanol, dans un récipient fermé, sous la pression propre du solvant, à des températures de 80 à 100°C.
